(19) Europäisches Patentamt / European Patent Office / Office européen des brevets

(11) **EP 3 839 051 A1**

(12) **EUROPEAN PATENT APPLICATION**

(43) Date of publication:
**23.06.2021  Bulletin 2021/25**

(21) Application number: **19218204.6**

(22) Date of filing: **19.12.2019**

(51) Int Cl.:
*C12P 7/40* (2006.01)          *C12P 13/10* (2006.01)
*C12N 9/10* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
**KH MA MD TN**

(71) Applicant: **Evonik Operations GmbH
45128 Essen (DE)**

(72) Inventors:
• **SCHNEIDER, Frank
  33790 Halle (DE)**
• **JANKOWITSCH, Frank
  48336 Sassenberg (DE)**

(74) Representative: **Evonik Patent Association
c/o Evonik Industries AG
IP Management
Bau 1042A/PB 15
Paul-Baumann-Straße 1
45772 Marl (DE)**

(54) **METHOD FOR THE FERMENTATIVE PRODUCTION OF GUANIDINOACETIC ACID**

(57)    The present invention relates to a microorganism transformed to be capable of producing guanidinoacetic acid (GAA) and to a method for the fermentative production of GAA using such microorganism. The present invention also relates to a method for the fermentative production of creatine.

EP 3 839 051 A1

**Description**

The present invention relates to a microorganism transformed to be capable of producing guanidinoacetic acid (GAA) and to a method for the fermentative production of GAA using such microorganism. The present invention also relates to a method for the fermentative production of creatine.

GAA is an organic compound used as animal feed additive (WO2005120246 A1/US2011257075 A1). GAA is a natural precursor of creatine (e.g. Humm et al., Biochem. J. (1997) 322, 771-776). Therefore, the supplementation of GAA allows for an optimal supply of creatine in the organism.

The present invention pertains to a method to produce GAA by a fermentative process using industrial feed stocks (e.g. ammonia, ammonium salts and glucose or sugar containing substrates) as starting material. In biological systems GAA and ornithine are formed from arginine and glycine as starting materials by the catalytic action of an L-arginine:glycine-amidinotransferase (AGAT; EC 2.1.4.1), which is the first step in creatine biosynthesis:

$$\text{L-arginine + glycine} \xrightarrow{\text{AGAT}} \text{L-ornithine + GAA}$$

Guthmiller et al. (J Biol Chem. 1994 Jul 1;269(26):17556-60) have characterized a rat kidney AGAT by cloning and heterologously expressing the enzyme in *E. coli.* Muenchhoff et al. (FEBS Journal 277 (2010) 3844-3860) report the first characterization of an AGAT from a prokaryote also by cloning and heterologously expressing the enzyme in *E. coli.* Sosio et al. (Cell Chemical Biology 25, 540-549, May 17, 2018) elucidated the biosynthetic pathway for pseudouridimycin in *Streptomyces* sp.. They describe as an intermediate reaction the formation of GAA and L-ornithine by the reaction of L-arginine with glycine catalyzed by PumN, an L-arginine:glycine-amidinotransferase (AGAT). Humm et al. expressed a recombinant gene encoding human AGAT in Escherichia coli and identified cysteine-407 as an active-site residue of AGAT (Biochem. J. (1997) 322, 771-776).

Several approaches for increasing the production of one of the starting materials in GAA synthesis, i.e. L-arginine, in microorganisms, particularly bacteria, are also known from literature. An overview for the metabolic engineering of *Corynebacterium glutamicum* (*C. glutamicum*) for L-arginine production is provided by Park et al. (NATURE COMMUNICATIONS | DOI: 10.1038/ncomms5618). They propose random mutagenesis and screening for L-arginine producers of already L-arginine producing *C. glutamicum* strains, e.g. of ATCC 21831 (Nakayama and Yoshida 1974, US3849250 A) and stepwise rational metabolic engineering based on system-wide analysis of metabolism results in a gradual increase in L-arginine production throughout the strain engineering steps. Yim et al. (J Ind Microbiol Biotechnol (2011) 38:1911-1920) could show that inactivation of the *argR*, gene coding for the central repressor protein ArgR controlling the L-arginine biosynthetic pathway, by disrupting the chromosomal *argR* gene in C. *glutamicum* leads to an improved arginine-producing strain. Ginesy et al. (Microbial Cell Factories (2015) 14:29) report the successful engineering of *E. coli* for enhanced arginine production. Among other, they proposed the deletion of the *argR* repressor gene.

Kurahashi et al. (EP1057893 A1) report on methods for increasing the L-arginine producing ability of a microorganism by enhancing L-arginine biosynthesis enzymes utilizing recombinant DNA techniques, e.g. by utilizing a microorganism belonging to the genus *Corynebacterium* or *Brevibacterium* which is made to harbor a recombinant DNA comprising a vector DNA and a DNA fragment containing genes for acetylornithine deacetylase, N-acetylglutamic acid- γ-semialdehyde dehydrogenase, N-acetyl glutamokinase and argininosuccinase derived from a microorganism belonging to the genus *Escherichia.* For an improved L-arginine production the authors further propose a microorganism which is enhanced in an activity of intracellular glutamate dehydrogenase (GDH) and which has an L-arginine producing ability.

A method of using a genetic recombinant strain, wherein a gene which inhibits the expression of arginine- biosynthesizing operon argR was inactivated has been reported by Suga et al. (US7160705 B2). In particular, the deletion in argR, which controls the arginine operon, has been considered as an important factor in arginine production.

In a microorganism of *Corynebacterium,* the *argCJBDFR* gene, which is involved in arginine biosynthesis, is constituted in the form of an operon and is subjected to feedback-inhibition by intracellular arginine (Sakanyan et al., Microbiology, 142:9-108, 1996), thus imposing a limitation on its high yield L-arginine production.

However, Bae et al. (EP3153573 A1) in an attempt to increase the production yield of L-arginine in *C. glutamicum*, discovered that L-arginine can be produced in higher yield compared to the parental L-arginine-producing strain, by enhancing the activities of the arginine operon and ornithine carbamoyltransferase (ArgF, ArgF2), without any deletion in arginine repressor (*argR*).

The arginine operon is an operon consisting of genes encoding enzymes involved in the mechanism of L- arginine biosynthesis, and in particular, arginine operon consists of genes encoding enzymes constituting the cyclic steps of L-arginine biosynthesis. Specifically, the arginine operon consists of N-acetylglutamyl phosphate reductase (ArgC), glutamate N-acetyltransferase (ArgJ), N-acetylglutamate kinase (ArgB), acetylornithine aminotransferase (ArgD), ornithine carbamoyltransferase (ArgF), and the arginine repressor (ArgR). These enzymes are involved in the continuous enzyme reactions of L-arginine biosynthesis.

Fan Wenchao discloses a method for the production of creatine by fermentation of non-pathogenic microorganisms, such as *Corynebacterium glutamicum* (CN106065411 A). The microorganism has the following biotransformation functions: glucose conversion to L-glutamic acid; conversion of L-glutamic acid to N-acetyl-L-glutamic acid; conversion of N-acetyl-L-glutamic acid to N-acetyl-L-glutamic acid semialdehyde; conversion of N-acetyl-L-glutamic acid semialdehyde to N- acetyl-L-ornithine; conversion of N-acetyl-L-ornithine to L-ornithine; conversion of L-ornithine to L-citrulline; conversion of L-citrulline to arginino-succinic acid; conversion of arginino-succinic acid to L-arginine; conversion of L-arginine to guanidinoacetic acid; and, finally, conversion of guanidinoacetic acid to creatine. Fan Wenchao proposes, that the microorganism overexpresses one or more enzymes selected from the group consisting of N-acetylglutamate-synthase, N-acetylornithine-δ-aminotransferase, N-acetylornithinase, ornithine-carbamoyl transferase, argininosuccinate synthetase, glycine amidino-transferase (EC: 2.1. 4.1), and guanidinoacetate N-methyltransferase (EC: 2.1.1.2). The microorganism overexpresses preferably glycine aminotransferase (L-arginine:glycine amidinotransferase) and guanidinoacetate N-methyltransferase.

Until now, microorganisms suitable for an increased production of GAA compared to their wildtype forms and a respective method for the production of GAA using such microorganisms have not been reported.

Therefore, the problem underlying the present invention is to provide a microorganism transformed to be capable of producing guanidinoacetic acid (GAA) and to a method for the fermentative production of GAA using such microorganism.

The problem is solved by a microorganism having an improved ability to produce L-arginine compared with the ability of the wildtype microorganism and/or having increased activities of an enzyme having the function of a carbamoylphosphate synthase (EC 6.3.4.16) compared to the respective enzymic activity in the wildtype microorganism and comprising at least one gene coding for a protein having the function of an L-arginine:glycine amidinotransferase.

Alternatively, the problem is solved by a microorganism having an improved ability to produce L-arginine compared with the ability of the wildtype microorganism and/or having increased activities of an enzyme having the function of an argininosuccinate lyase (E.C. 4.3.2.1) compared to the respective enzymic activity in the wildtype microorganism and comprising at least one gene coding for a protein having the function of an L-arginine:glycine amidinotransferase.

Proteins having the function of an L-arginine:glycine amidinotransferase (AGAT) belong to the amidinotransferase family. The amidinotransferase family comprises glycine (EC:2.1.4.1) and inosamine (EC:2.1.4.2) amidinotransferases, enzymes involved in creatine and streptomycin biosynthesis respectively. This family also includes arginine deiminases, EC:3.5.3.6. These enzymes catalyse the reaction: arginine + H2O <=> citrulline + NH3. Also found in this family is the Streptococcus anti tumour glycoprotein. Enzymes or proteins with an L-arginie:glycine-amidinotransferase (AGAT) activity are also described to possess a conserved domain that belongs to the PFAM Family: Amidinotransf (PF02274) (: Marchler-Bauer A et al. (2017), "CDD/SPARCLE: functional classification of proteins via subfamily domain architectures.", Nucleic Acids Res. 45(D1):D200-D203.) as described also in the following publications: Pissowotzki K et al. , Mol Gen Genet 1991;231:113-123 (PUBMED:1661369 EPMC:1661369); D'Hooghe I et al., J Bacteriol 1997;179:7403-7409 (PUBMED:9393705 EPMC:9393705); Kanaoka M et al. , Jpn J Cancer Res 1987;78:1409-1414 (PUBMED:3123442 EPMC:3123442).

The activity of an enzyme having the function of an argininosuccinate lyase (E.C. 4.3.2.1) in the microorganism according to the present invention may be increased compared to the respective enzymic activity in the wildtype microorganism.

Alternatively, the activity of an enzyme having the function of a carbamoylphosphate synthase (EC 6.3.4.16) in the microorganism according to the present invention may be increased compared to the respective enzymic activity in the wildtype microorganism.

Furthermore, in the microorganism according to the present invention, the activity of an enzyme having the function of an ornithine carbamoyltransferase (EC 2.1.3.3) may be increased compared to the respective enzymic activity in the wildtype microorganism.

In the microorganism according to the present invention, the activity of an enzyme having the function of an argininosuccinate synthetase (E.C. 6.3.4.5) may be also increased compared to the respective enzymic activity in the wildtype microorganism.

Increased enzyme activities in microorganisms can be achieved, for example, by mutation of the corresponding endogenous gene. A further measure to increase enzymic activities may be to stabilize the mRNA coding for the enzymes.

The increased activities of the above-mentioned enzymes may also be achieved by overexpressing the genes coding for the respective enzymes. In other words, the problem is preferably solved by a microorganism having an improved ability to produce L-arginine compared with the ability of the wildtype organism and/or having at least one or more overexpressed genes (e.g. carA, carB) coding for a protein having the function of a carbamoylphosphate synthase (EC 6.3.4.16), and further comprising a gene coding for a protein having the function of an L-arginine:glycine amidinotransferase (AGAT, e.g. EC 2.1.4.1).

The microorganism according to the present invention preferably also comprises at least one or more overexpressed genes selected from the group consisting of a gene (e.g. *argF/argF2/argI)* coding for a protein having the function of an ornithine carbamoyltransferase (EC 2.1.3.3), a gene (e.g. *argG)* coding for a protein having the function of an argininosuccinate synthetase (E.C. 6.3.4.5), and a gene (e.g. *argH)* coding for a protein having the function of an argininosuccinate lyase (E.C. 4.3.2.1).

Overexpression of a gene is generally achieved by increasing the copy number of the gene and/or by functionally linking the gene with a strong promoter and/or by enhancing the ribosomal binding site and/or by codon usage optimization of the start codon or of the whole gene or a combination comprising a selection of or all methods mentioned above.

In the context of the present invention, a microorganism having an improved ability to produce L-arginine means a microorganism producing L-arginine in excess of its own need. Examples for such L-arginine producing microorganisms are e.g. *C. glutamicum* ATCC 21831 or those disclosed by Park et al. (NATURE COMMUNICATIONS | DOI: 10.1038/ncomms5618) or by Ginesy et al. (Microbial Cell Factories (2015) 14:29).

The microorganism according to the present invention is preferably recombinant and the gene coding for the protein having the function of an L-arginine:glycine amidinotransferase (AGAT) is heterologous.

A heterologous gene means that the gene has been inserted into a host organism which does not naturally have this gene. Insertion of the heterologous gene in the host is performed by recombinant DNA technology. Microorganisms that have undergone recombinant DNA technology are called transgenic, genetically modified or recombinant.

In one embodiment of the present invention, the *argR* gene coding for the arginine responsive repressor protein ArgR in the microorganism according to the present invention is neither attenuated nor deleted. Instead, in the microorganism according to the present invention the activity of the arginine responsive repressor protein ArgR may be increased compared to the respective activity in the wildtype microorganism.

Furthermore, in the microorganism according to the present invention the arginine operon (*argCJBDFR*) may be overexpressed.

Alternatively, in the microorganism according to the present invention the *argR* gene coding for the arginine responsive repressor protein ArgR may be attenuated or deleted.

In a further embodiment of the present invention and, optionally in addition to the above-mentioned modifications, at least one or more of the genes coding for an enzyme of the biosynthetic pathway of L-arginine, comprising of *gdh, argJ, argB, argC* and/or *argD* coding for a glutamate dehydrogenase, an ornithine acetyltransferase, an acetylglutamate kinase, an acetylglutamylphosphate reductase and an acetylornithine aminotransferase, respectively, is overexpressed in the microorganism according to the present invention.

Table 1 shows the different names of enzymes involved in or contributing to arginine biosynthesis in different species, i.e. *E. coli, C. glutamicum* and *Pseudomonas putida* (*P. putida*).

In the microorganism of the present invention the gene coding for a protein having the function of an L-arginine:glycine amidinotransferase may further be overexpressed. Overexpression of a gene is generally achieved by increasing the copy number of the gene and/or by functionally linking the gene with a strong promoter and/or by enhancing the ribosomal binding site and/or by codon usage optimization of the start codon or of the whole gene or a combination comprising a selection or all methods mentioned above.

Table 1: Names of Enzymes

| Enzyme name | Alias | EC Number | *E. coli* | *C. glutamicum* | *P. putida* |
|---|---|---|---|---|---|
| glutamate dehydrogenase | NADP-SPECIFIC GLUTAMATE DEHYDROGENASE | 1.4.1.4 (1.4.1.2 in P. putida) | gdhA | gdh | gdhA/ gdhB |
| N-acetyl glutamokinase | ACETYLGLUTAMATE KINASE | 2.7.2.8 | argB | argB | argB |
| N-acetylglutamyl phosphate reductase | N-acetyl-gamma-glutamylphosphate reductase | 1.2.1.38 | argC | argC | argC |
| N-acetylglutamic acid-γ-semialdehyde dehydrogenase | ACETYLORNITHINE AMINOTRANSFERASE (in e.c. bifunctional acetylornithine aminotransferase/ succinyldiaminopimelate aminotransferase) | 2.6.1.11/ 2.6.1.17 | argD | argD | aruC |
| acetylornithine deacetylase | bifunctional acetylornithine deacetylase / GLUTAMATE N-ACETYLTRANSFERASE | 2.3.1.35/ 2.3.1.1 | | argJ | argJ |

(continued)

| Enzyme name | Alias | EC Number | *E. coli* | *C. glutamicum* | *P. putida* |
|---|---|---|---|---|---|
| acetylornithine deacetylase | | 3.5.1.16 | argE | | argE |
| | GLUTAMATE N-ACETYLTRANSFERASE | 2.3.1.1 | argA | | argA |
| ornithine carbamoyltransferase | ornithine carbamoyltransferase 1 | 2.1.3.3 | argI | argF/argF2 | arcB/argF |
| Argininosuccinate synthetase | ARGININOSUCCINATE SYNTHASE | 6.3.4.5 | argG | argG | argG |
| Argininosuccinate lyase | ARGININOSUCCINATE LYASE | 4.3.2.1 | argH | argH | argH |
| carbamoyl-phosphate synthase | carbamoyl-phosphate synthase | 6.3.5.5 | carAB | carAB | carAB |
| carbamate kinase | carbamate kinase | 2.7.2.2 | ybcF/yqeA | | arcC |

The protein having the function of an L-arginine:glycine amidinotransferase in the microorganism of the present invention may comprise an amino acid sequence which is at least 80 % homologous, preferably at least 90 % homologous to the amino acid sequence according to SEQ ID NO: 4. In a further embodiment of the present invention the amino acid sequence of the L-arginine:glycine amidinotransferase is identical to amino acid sequence according to SEQ ID NO: 4.

The microorganism of the present invention may belong to the genus *Corynebacterium,* preferably *Corynebacterium glutamicum* (*C. glutamicum*), or to the genus *Enterobacteriaceae,* preferably *Escherichia coli* (*E. coli*), or to the genus *Pseudomonas*, preferably *Pseudomonas putida (P. putida)*.

Generally, increased enzyme activities in microorganisms can be achieved, for example, by mutation of the corresponding endogenous gene. Enzyme activities can also be enhanced by overexpression of the corresponding gene.

Generally, the overexpression of a gene, according to the present invention, is achieved by increasing the copy number of the gene and/or by an enhancement of regulatory factors, e.g. by functionally linking the gene with a strong promoter and/or by enhancing the ribosomal binding site and/or by codon usage optimization of the start codon or of the whole gene. The enhancement of such regulatory factors which positively influence gene expression can, for example, be achieved by modifying the promoter sequence upstream of the structural gene in order to increase the effectiveness of the promoter or by completely replacing said promoter with a more effective or a so-called strong promoter. Promoters are located upstream of the gene. A promoter is a DNA sequence consisting of about 40 to 50 base pairs and which constitutes the binding site for an RNA polymerase holoenzyme and the transcriptional start point, whereby the strength of expression of the controlled polynucleotide or gene can be influenced. Generally, it is possible to achieve an overexpression or an increase in the expression of genes in bacteria by selecting strong promoters, for example by replacing the original promoter with strong, native (originally assigned to other genes) promoters or by modifying certain regions of a given, native promoter (for example its so-called -10 and -35 regions) towards a consensus sequence, e.g. as taught by M. Patek et al. (Microbial Biotechnology 6 (2013), 103-117) for *C. glutamicum.* A "functional linkage" is understood to mean the sequential arrangement of a promoter with a gene, which leads to a transcription of the gene.

The genetic code is degenerated which means that a certain amino acid may be encoded by a number of different triplets. The term codon usage refers to the observation that a certain organism will typically not use every possible codon for a certain amino acid with the same frequency. Instead an organism will typically show certain preferences for specific codons meaning that these codons are found more frequently in the coding sequence of transcribed genes of an organism. If a certain gene foreign to its future host, i.e. from a different species, should be expressed in the future host organism the coding sequence of said gene should then be adjusted to the codon usage of said future host organism (i.e. codon usage optimization).

The above-mentioned problem is further solved by a method for the fermentative production of guanidino acetic acid (GAA), comprising the steps of a) cultivating the microorganism according to the present invention as defined above in a suitable medium under suitable conditions, and b) accumulating GAA in the medium to form an GAA containing fermentation broth.

The method according to the present invention may further comprise adding glycine and/or adding L-arginine and/or adding L-ornithine to the medium. Preferably, the medium is supplemented with glycine in a concentration ranging from 0.1 to 300 g glycine/l medium, preferably 0.82 g glycine/l medium, and/or with L-arginine to obtain a concentration ranging from 0.1 to 200 g L-arginine/l medium, preferably 1.9 g L-arginine/l medium.

The method of the present invention may further comprise the step of isolating GAA from the fermentation broth.

The method according to the present invention may further comprise the step of drying and/or granulating the GAA containing fermentation broth.

The present invention further concerns a microorganism as defined above, further comprising a gene coding for an enzyme having the activity of a guanidinoacetate N-methyltransferase (EC: 2.1.1.2). Preferably, the gene coding for an enzyme having the activity of a guanidinoacetate N-methyltransferase is overexpressed.

The present invention also concerns a method for the fermentative production of creatine, comprising the steps of a) cultivating the microorganism according to the present invention comprising a gene coding for an enzyme having the activity of a guanidinoacetate N-methyltransferase in a suitable medium under suitable conditions, and b) accumulating creatine in the medium to form a creatine containing fermentation broth.

Preferably, the method further comprises isolating creatine from the creatine containing fermentation broth. creatine may be be extracted from fermentation broth by isoelectric point method and / or ion exchange method. Alternatively, creatine can be further purified by a method of recrystallization in water.

## EXPERIMENTAL SECTION

### A) MATERIALS and METHODS

### Chemicals

Kanamycin solution from *Streptomyces kanamyceticus* was purchased from Sigma Aldrich (St. Louis, USA, Cat. no. K0254). IPTG (Isopropyl β-D-1-thiogalactopyranoside) was purchased from Carl-Roth (Karlsruhe, Germany, Cat. no. 2316.4.). If not stated otherwise, all other chemicals were purchased analytically pure from Merck (Darmstadt, Germany), Sigma Aldrich (St. Louis, USA) or Carl-Roth (Karlsruhe, Germany).

### Cultivation for cell proliferation

If not stated otherwise, cultivation / incubation procedures were performed as follows herewith:

a. LB broth (MILLER) from Merck (Darmstadt, Germany; Cat. no. 110285) was used to cultivate *E. coli* strains in liquid medium. The liquid cultures (10 ml liquid medium per 100 ml Erlenmeyer flask with 3 baffles) were incubated in the Infors HT Multitron standard incubator shaker from Infors GmbH (Bottmingen, Switzerland) at 30°C and 200 rpm.

b. LB agar (MILLER) from Merck (Darmstadt, Germany, Cat. no. 110283) was used for cultivation of *E. coli* strains on agar plates. The agar plates were incubated at 30°C in an INCU-Line® mini incubator from VWR (Radnor, USA).

c. Brain heart infusion broth (BHI) from Merck (Darmstadt, Germany, Cat. no. 110493) was used to cultivate *C. glutamicum* strains in liquid medium. The liquid cultures (10 ml liquid medium per 100 ml Erlenmeyer flask with 3 baffles) were incubated in the Infors HT Multitron standard incubator shaker from Infors GmbH (Bottmingen, Switzerland) at 30°C and 200 rpm.

d. Brain heart agar (BHI-agar) from Merck (Darmstadt, Germany, Cat. no. 113825) was used for cultivation of *C. glutamicum* strains on agar plates. The agar plates were incubated at 30°C in an incubator from Heraeus Instruments with Kelvitron® temperature controller (Hanau, Germany).

e. For cultivating *C. glutamicum* after electroporation, BHI-agar (Merck, Darmstadt, Germany, Cat. no. 113825) was supplemented with 134 g/l sorbitol (Carl Roth GmbH + Co. KG, Karlsruhe, Germany), 2.5 g/l yeast extract (Oxoid/ThermoFisher Scientific, Waltham, USA, Cat. no. LP0021) and 25 mg/l kanamycin. The agar plates were incubated at 30°C in an incubator from Heraeus Instruments with Kelvitron® temperature controller (Hanau, Germany).

### Determining optical density of bacterial suspensions

a. The optical density of bacterial suspensions in shake flask cultures was determined at 600 nm (OD600) using the BioPhotometer from Eppendorf AG (Hamburg, Germany).

b. The optical density of bacterial suspensions produced in the Wouter Duetz (WDS) micro fermentation system (24-Well Plates) was determined at 660 nm (OD660) with the GENios™ plate reader from Tecan Group AG (Männedorf, Switzerland).

**Centrifugation**

a. Bacterial suspensions with a maximum volume of 2 ml were centrifuged in 1.5 ml or 2 ml reaction tubes (e.g. Eppendorf Tubes® 3810X) using an Eppendorf 5417 R benchtop centrifuge (5 min. at 13.000 rpm).
b. Bacterial suspensions with a maximum volume of 50 ml were centrifuged in 15 ml or 50 ml centrifuge tubes (e.g. FalconTM 50 ml Conical Centrifuge Tubes) using an Eppendorf 5810 R benchtop centrifuge for 10 min. at 4.000 rpm.

**DNA isolation**

Plasmid DNA was isolated from *E. coli* cells using the QIAprep Spin Miniprep Kit from Qiagen (Hilden, Germany, Cat. No. 27106) according to the instructions of the manufacturer.

**Polymerase chain reaction (PCR)**

PCR with a proof reading (high fidelity) polymerase was used to amplify a desired segment of DNA for Sanger sequencing or DNA assembly. Non-proof-reading polymerase Kits were used for determining the presence or absence of a desired DNA fragment directly from *E. coli* or *C. glutamicum* colonies.
a. The Phusion® High-Fidelity DNA Polymerase Kit (Phusion Kit) from New England BioLabs Inc. (Ipswich, USA, Cat. No. M0530) was used for template-correct amplification of selected DNA regions according to the instructions of the manufacturer (see table 2).

Table 2: Thermocycling conditions for PCR with Phusion® High-Fidelity DNA Polymerase Kit from New England BioLabs Inc.

| PCR Program | | | |
|---|---|---|---|
| Step | Time [min.:sec.] | T [°C] | Description |
| 1 | 00:30 | 98 | Initial denaturation step |
| 2 | 00:05 | 98 | Denaturation step |
| 3 | 00:30 | 60 | Annealing step |
| 4 | 00:xx | 72 | Elongation step 1 min. per kb DNA |
| | | | Repeat step 2 to 4: 35 x |
| 5 | 05:00 | 72 | Final elongation step |
| 6 | Hold | 4 | Cooling step |

b. Taq PCR Core Kit (Taq Kit) from Qiagen (Hilden, Germany, Cat. No.201203) was used to amplify a desired segment of DNA in order to confirm its presence. The kit was used according to the instructions of the manufacturer (see table 3).

Table 3: Thermocycling conditions for PCR with Taq PCR Core Kit (Taq Kit) from Qiagen.

| PCR Program | | | |
|---|---|---|---|
| Step | Time [min.:sec.] | T [°C] | Description |
| 1 | 05:00 | 94 | Initial denaturation step |
| 2 | 00:30 | 94 | Denaturation step |
| 3 | 00:30 | 52 | Annealing step |
| 4 | 01:20 | 72 | Elongation step 1 min. per kb DNA |
| | | | Repeat step 2 to 4: 35 x |

(continued)

| PCR Program | | | |
|---|---|---|---|
| Step | Time [min.:sec.] | T [°C] | Description |
| 5 | 04:00 | 72 | Final elongation step |
| 6 | Hold | 4 | Cooling step |

c. SapphireAmp® Fast PCR Master Mix (Sapphire Mix) from Takara Bio Inc (Takara Bio Europe S.A.S., Saint-Germain-en-Laye, France, Cat. No. RR350A/B) was used as an alternative to confirm the presence of a desired segment of DNA in cells taken from *E. coli* or *C. glutamicum* colonies according to the instructions of the manufacturer (see table 4).

Table 4: Thermocycling conditions for PCR with SapphireAmp® Fast PCR Master Mix (Sapphire Mix) from Takara Bio Inc.

| PCR Program | | | |
|---|---|---|---|
| Step | Time [min.:sec.] | T [°C] | Description |
| 1 | 01:00 | 94 | Initial denaturation step |
| 2 | 00:05 | 98 | Denaturation step |
| 3 | 00:05 | 55 | Annealing step |
| 4 | 00:05 | 72 | Elongation step |
| | | | Repeat step 2 to 4: 30 x |
| 5 | 04:00 | 72 | Final elongation step |
| 6 | Hold | 4 | Cooling step |

d. All oligonucleotide primers were synthesized by Eurofins Genomics GmbH (Ebersberg, Germany) using the phosphoramidite method described by McBride and Caruthers (1983).

e. As PCR template either a suitably diluted solution of isolated plasmid DNA or of total DNA isolated from a liquid culture or the total DNA contained in a bacterial colony (colony PCR) was used. For said colony PCR the template was prepared by taking cell material with a toothpick from a colony on an agar plate and placing the cell material directly into the PCR reaction tube. The cell material was heated for 10 sec. with 800 W in a microwave oven type Mikrowave & Grill from SEVERIN Elektrogeräte GmbH (Sundern, Germany) and then the PCR reagents were added to the template in the PCR reaction tube.

f. All PCR reactions were carried out in PCR cyclers type Mastercycler or Mastercycler nexus gradient from Eppendorf AG (Hamburg, Germany).

**Restriction enzyme digestion of DNA**

For restriction enzyme digestions either "FastDigest restriction endonucleases (FD)" (ThermoFisher Scientific, Waltham, USA) or restriciton endonucleases from New England BioLabs Inc. (Ipswich, USA) were used. The reactions were carried out according to the instructions of the manufacturer's manual.

**Determining the sizes of DNA fragments**

a. The sizes of small DNA fragments (<1000 bps) were usually determined by automatic capillary electrophoresis using the QIAxcel from Qiagen (Hilden, Germany).

b. If DNA fragments needed to be isolated or if the DNA fragments were >1000 bps DNA was separated by TAE agarose gel electrophoresis and stained with GelRed® Nucleic Acid Gel Stain (Biotium, Inc., Fremont, Canada). Stained DNA was visualized at 302 nm.

**Purification of PCR amplificates and restriction fragments**

PCR amplificates and restriction fragments were cleaned up using the QIAquick PCR Purification Kit from Qiagen (Hilden, Germany; Cat. No. 28106), according to the manufacturer's instructions. DNA was eluted with 30 μl 10 mM Tris*HCl (pH 8.5).

**Determining DNA concentration**

DNA concentration was measured using the NanoDrop Spectrophotometer ND-1000 from PEQLAB Biotechnologie GmbH, since 2015 VWR brand (Erlangen, Germany).

**Assembly cloning**

Plasmid vectors were assembled using the "NEBuilder HiFi DNA Assembly Cloning Kit" purchased from New England BioLabs Inc. (Ipswich, USA, Cat. No. E5520). The reaction mix, containing the linear vector and at least one DNA insert, was incubated at 50°C for 60 min.. 0.5 μl of Assembly mixture was used for each transformation experiment.

**Chemical transformation of *E. coli***

For plasmid cloning, chemically competent "NEB® Stable Competent *E. coli* (High Efficiency)" (New England BioLabs Inc., Ipswich, USA, Cat. No. C3040) were transformed according to the manufacturer's protocol. Successfully transformed cells were selected on LB agar supplemented with 25 mg/l kanamycin.

**Transformation of *C. glutamicum***

Transformation of *C. glutamicum* with plasmid-DNA was conducted via electroporation using a "Gene Pulser Xcell" (Bio-Rad Laboratories GmbH, Feldkirchen, Germany) as described by Ruan *et al.* (2015). Electroporation was performed in 1 mm electroporation cuvettes (Bio-Rad Laboratories GmbH, Feldkirchen, Germany) at 1.8 kV and a fixed time constant set to 5 ms. Transformed cells were selected on BHI-agar containing 134 g/l sorbitol, 2.5 g/l Yeast Extract and 25 mg/l kanamycin.

**Determining nucleotide sequences**

Nucleotide sequences of DNA molecules were determined by Eurofins Genomics GmbH (Ebersberg, Germany) by cycle sequencing, using the dideoxy chain termination method of Sanger et al. (Proceedings of the National Academy of Sciences USA 74, 5463 - 5467, 1977), on Applied Biosystems® (Carlsbad, CA, USA) 3730xl DNA Analyzers. Clonemanager Professional 9 software from Scientific & Educational Software (Denver, USA) was used to visualise and evaluate the sequences.

**Glycerol stocks of *E. coli* and *C. glutamicum* strains**

For long time storage of *E. coli-* and *C. glutamicum* strains glycerol stocks were prepared. Selected *E. coli* clones were cultivated in 10 ml LB medium supplemented with 2 g/l glucose. Selected *C. glutamicum* clones were cultivated in 10 ml twofold concentrated BHI medium supplemented with 2 g/l glucose. Cultures of plasmid containing *E. coli-* and *C. glutamicum* strains were supplemented with 25 mg/l kanamycin. The medium was contained in 100 ml Erlenmeyer flasks with 3 baffles. It was inoculated with a loop of cells taken from a colony. The culture was then incubated for 18 h at 30°C and 200 rpm. After said incubation period 1.2 ml 85 % (v/v) sterile glycerol were added to the culture. The obtained glycerol containing cell suspension was then aliquoted in 2 ml portions and stored at -80°C.

**GAA production in millilitre-scale cultivations**

The millilitre-scale cultivation system according to Duetz (2007) was used to assess the GAA-production of the strains. For this purpose, 24-deepwell microplates (24 well WDS plates) from EnzyScreen BV (Heemstede, Netherlands, Cat. no. CR1424) filled with 2.5 ml medium per well were used.

Precultures of the strains were done in 10 ml seed medium (SM). The medium was contained in a 100 ml Erlenmeyer flask with 3 baffles. It was inoculated with 100 μl of a glycerol stock culture and the culture incubated for 24 h at 30°C and 200 rpm. The composition of the seed medium (SM) is shown in table 5.

Table 5: Seed medium (SM)

| Components | Concentration (g/l) |
|---|---|
| Yeast extract FM902 (Angel Yeast Co.,LTD, Hubei, P.R.China) | 10 |
| Urea | 1.5 |
| $KH_2PO_4$ | 0.5 |
| $K_2HPO_4$ | 0.5 |
| $MgSO_4 * 7 H_2O$ | 1 |
| Biotin | 0.0001 |
| Thiamine hydrochloride | 0.0001 |
| $FeSO_4 * 7 H_2O$ | 0.01 |
| $MnSO_4 * H_2O$ | 0.01 |
| Glucose | 20 |
| pH=7.0 | |

After said incubation period the optical densities OD600 of the precultures were determined. The volume, needed to inoculate 2.5 ml of production medium (PM) to an OD600 of 0.1, was sampled from the preculture, centrifuged (1 min at 8000 g) and the supernatant was discarded. Cells were then resuspended in 100 µl of production medium.

The main cultures were started by inoculating the 2.4 ml production medium (PM) containing wells of the 24 Well WDS-Plate with each 100 µl of the resuspended cells from the precultures. The composition of the production medium (PM) is shown in table 6.

Table 6: Production medium (PM)

| Components | Concentration (g/l) |
|---|---|
| 3-(N-morpholino)propanesulfonic acid (MOPS) | 40 |
| Yeast extract FM902 (Angel Yeast Co.,LTD, Hubei, P.R.China) | 1.5 |
| $(NH_4)_2SO_4$ | 10 |
| $NH_4Cl$ | 15 |
| Trisodium citrate * 2 $H_2O$ | 10 |
| Urea | 1 |
| $KH_2PO_4$ | 0.5 |
| $K_2HPO_4$ | 0.5 |
| $MgSO_4 * 7 H_2O$ | 1 |
| Biotin | 0.0001 |
| Thiamine hydrochloride | 0.0001 |
| $FeSO_4 * 7 H_2O$ | 0.01 |
| $MnSO_4 * H_2O$ | 0.01 |
| $ZnSO_4 * 7 H_2O$ | 0.000015 |
| $CuSO_4 * 5 H_2O$ | 0.0004 |
| Antifoam XFO-1501 (Ivanhoe Industries Inc., Zion, USA) | 0.5 |
| Glucose | 40 |
| IPTG (Isopropyl β-D-1-thiogalactopyranoside) | 0.3 mM |
| pH=7.2 | |

The main cultures were incubated for 72 h at 30 °C and 300 rpm in an Infors HT Multitron standard incubator shaker from Infors GmbH (Bottmingen, Switzerland) until complete consumption of glucose. The glucose concentration in the suspension was analysed with the blood glucose-meter OneTouch Vita® from LifeScan (Johnson & Johnson Medical GmbH, Neuss, Germany).

After cultivation the culture suspensions were transferred to a deep well microplate. A part of the culture suspension was suitably diluted to measure the OD600. Another part of the culture was centrifuged and the concentration of GAA in the supernatant was analyzed as described below.

**Determination of L-arginine and glycine content in yeast pepton FM902**

As yeast extract FM902 (Angel Yeast Co.,LTD, Hubei, P.R.China) contains various peptides and amino acids, its content of L-arginine and glycine was measured as follows.

For measuring free amino acids, the samples were prepared by dissolving 1 g of yeast extract in 20 ml of water. The solution was filled up with water to a total volume of 25 ml, mixed thoroughly and filtered using a 0.2 $\mu$M nylon syringe filter.

For measuring total amino acids (free amino acids plus amino acids bound in peptides), the samples were prepared by dissolving 1 g yeast extract in 10 ml 6M HCl and incubating them for 24h at 110°C. Then, water was added up to a total volume of 25 ml. The solution was mixed thoroughly and filtered using a 0.2 $\mu$M nylon syringe filter.

The concentrations of L-arginine and glycine in the samples were determined by ion exchange chromatography using a SYKAM S433 amino acid analyzer from SYKAM Vertriebs GmbH (Fürstenfeldbruck, Germany). As solid phase a column with spherical, polystyrene-based cation exchanger (Peek LCA N04/Na, dimension 150 x 4.6 mm) from SYKAM was used. Depending on the L-amino acid the separation takes place in an isocratic run using a mixture of buffers A and B for elution or by gradient elution using said buffers. As buffer A an aqueous solution containing in 20 l 263 g trisodium citrate, 120 g citric acid, 1100 ml methanol, 100 ml 37 % HCl and 2 ml octanoic acid (final pH 3.5) was used. As buffer B an aqueous solution containing in 20 l 392 g trisodium citrate, 100 g boric acid and 2 ml octanoic acid (final pH 10.2) was used. The free amino acids were coloured with ninhydrin through post-column derivatization and detected photometrically at 570 nm.

Table 7 shows the content of free and total L-arginine and glycine determined in yeast extract FM902 (Angel Yeast Co.,LTD, Hubei, P.R.China), as well as the resulting amounts in the production medium (PM).

Table 7: Content of L-arginine and glycine in yeast extract (YE) FM902 and resulting concentrations in production medium (PM) containing 1.5 g/l YE.

| Amino acid | Free amino acid in YE | Total amino acid in YE | Resulting free amino acid in PM | Resulting total amino acid in PM |
|---|---|---|---|---|
| L-arginine | 15.1 g/kg | 32.1 g/kg | 22.7 mg/l | 48.2 mg/l |
| glycine | 6.9 g/kg | 30.5 g/kg | 10.4 mg/l | 45.7 mg/l |

**Quantification of GAA**

Samples were analyzed with an analyzing system from Agilent, consisting of a HPLC "Infinity 1260" coupled with a mass analyzer "Triple Quad 6420" (Agilent Technologies Inc., Santa Clara, USA). Chromatographic separation was done on the Atlantis HILIC Silica column, 4,6X250mm, 5$\mu$m (Waters Corporation, Milford, USA) at 35°C. Mobile phase A was water with 10mM ammonium formate and 0,2% formic acid. Mobile phase B was a mixture of 90% acetonitrile and 10 % water, 10 mM ammonium formate were added to the mixture. The HPLC system was started with 100% B, followed by a linear gradient for 22 min and a constant flow rate of 0,6 mL/min to 66% B. The mass analyzer was operated in the ESI positive ionization mode. For detection of GAA the m/z values were monitored by using an MRM fragmentation [M+H] + 118 - 76. The limit of quantification (LOQ) for GAA was fixed to 7 ppm.

**B) EXPERIMENTAL RESULTS**

**Example 1: Cloning of a gene putatively coding for an L-arginine:glycine amidinotransferase (AGAT, EC 2.1.4.1) from *Moorea producens***

*Moorea producens* is a filamentous cyanobacterium. The genome of the *Moorea producens* strain PAL-8-15-08-1 was published By Leao et al. (Leao T, Castelão G, Korobeynikov A, Monroe EA, Podell S, Glukhov E, Allen EE, Gerwick WH, Gerwick L, Proc Natl Acad Sci U S A. 2017 Mar 21;114(12):3198-3203. doi: 10.1073/pnas.1618556114;

Genbank accession Number CP017599.1). It contains an open reading frame putatively coding for a L-arginine:glycine amidinotransferase (AGAT, EC 2.1.4.1; locus_tag BJP34_00300 shown in SEQ ID NO:1). SEQ ID NO:2 shows the derived amino acid sequence (Genbank accession Number WP_070390602).

Using the software tool "GeneOptimizer" (Geneart/ ThermoFisher Scientific, Waltham, USA) this amino acid sequence was translated back into a DNA sequence optimized for the codon usage of *C. glutamicum.* Its ends were expanded with sequences for assembly cloning and 5 base pairs upstream of the open reading frame a Shine-Dalgarno-Sequenz (AGGA) was added. The resulting DNA sequence (SEQ ID NO:3) was ordered for gene synthesis from Invitrogen/Geneart (Thermo Fisher Scientific, Waltham, USA) and it was delivered as part of a cloning plasmid (designated as pMA-T_AGAT_Mp).

**Example 2: Cloning of AGAT_Mp into the expression plasmid pEC-XK99E**

The *E. coli-C. glutamicum* shuttle plasmid pEC-XK99E was digested using the restriction endonuclease SmaI. Terminal phosphates were removed using the "FastAP Thermosensitive Alkaline Phosphatase" (Thermo Fisher Scientific, Waltham, USA). The DNA was then purified with the "QIAquick PCR Purification Kit" (Qiagen GmbH, Hilden, Germany).

The cloning plasmid pMA-T_AGAT_Mp was digested with MluI + AatII and the resulting fragments were blunted using the "Fast DNA End Repair Kit" (Thermo Fisher Scientific, Waltham, USA). They were separated by agarose gel electrophoresis (0,8% agarose in TAE buffer) and the band corresponding to "AGAT_Mp" (1174 bp) was cut out. Its DNA was purified using the "QIAquick Gel Extraction Kit" (Qiagen GmbH, Hilden, Germany).

The AGAT_Mp fragment and the linearized pEC-XK99E were ligated using the "Ready-To-Go T4 DNA ligase" (GE Healthcare Europe GmbH, Freiburg, Germany). The ligation product was transformed into "NEB Stable Competent *E. coli* (High Efficiency)" (New England Biolabs, Ipswich, USA) and the cells were grown on LB agar containing 25 mg/l kanamycin. Appropriate clones were identified by restriction enzyme digestion and DNA sequencing. The resulting plasmid was named pEC-XK99E_AGAT_Mp.

**Example 3: Cloning of the gene *argF* into plasmid pCR-Blunt II-TOPO**

The *argF* gene was PCR amplified with the Phusion High-Fidelity DNA Polymerase Kit (New England BioLabs Inc., Ipswich, USA) using genomic DNA of *C. glutamicum* ATCC13032 and the oligonucleotide primer argF_1.p (SEQ ID NO:5) and argF_2.p (SEQ ID NO:6). The resulting PCR product was cloned into the plasmid pCR-Blunt II-TOPO (Thermo Fisher Scientific/Invitrogen, Waltham, USA) and a proper plasmid clone was identified by restriction enzyme digestion and DNA sequencing. This plasmid was named **pCRII-argF.**

**Example 4: Cloning of the genes *argG* and *argH* into plasmid pCR-Blunt II-TOPO**

The genes *argG* and *argH* were PCR amplified with the Phusion High-Fidelity DNA Polymerase Kit (New England BioLabs Inc., Ipswich, USA) using genomic DNA of *C. glutamicum* ATCC13032 and the oligonucleotide primer argG_1.p (SEQ ID NO:7) and argH_2.p (SEQ ID NO:8). The resulting PCR product was cloned into the plasmid pCR-Blunt II-TOPO (Thermo Fisher Scientific/Invitrogen, Waltham, USA) and a proper plasmid clone was identified by restriction enzyme digestion and DNA sequencing. The plasmid was named pCRII-argGH.

**Example 5: Cloning of the genes *argG* and *argH* into plasmid pCRII-argF**

pCRII-argGH was cut using HpaI + AvrII and a restriction fragment of 2773 bps was isolated from an agarose gel. pCRII-argF was cut using SspI + AvrII and a restriction fragment of 4526 bps was isolated from an agarose gel. Both fragments were ligated and then transformed into *E. coli.* Proper plasmid clones were identified by restriction enzyme digestion and DNA sequencing. The resulting plasmid was named pCRII-argFGH.

**Example 6: Cloning of the genes *argF, argG* and *argH* into the expression plasmid pEC-XK99E**

pCRII-argFGH was cut using HpaI + AvrII and a restriction fragment of 2773 bps was isolated from an agarose gel. The plasmid pEC-XK99E was cut using Ecl136II + XbaI. A restriction fragment of 6999 bps was isolated from an agarose gel. Both fragments were ligated and then transformed into *E. coli.* Proper plasmid clones were identified by restriction digestion and DNA sequencing. The resulting plasmid pEC-XK99E_argFGH contains the genes *argF, argG* and *argH* from *C. glutamicum.*

### Example 7: Cloning of the genes *argF*, *argG* and *argH* into the expression plasmid pEC-XK99E_AGAT_Mp

pCRII-argFGH was cut using Xbal + Spel and a restriction fragment of 3868 bps was isolated from an agarose gel. The plasmid pEC-XK99E_AGAT_Mp was cut using Xbal. A restriction fragment of 8188 bps was isolated from an agarose gel. Both fragments were ligated and then transformed into *E. coli*. Proper plasmid clones were identified by restriction digestion and DNA sequencing. The resulting plasmid pEC-XK99E_AGAT_Mp_argFGH contains the genes *argF*, *argG* and *argH* from *C. glutamicum* in combination with AGAT_Mp.

### Example 8: Cloning of the gene *argF* into the expression plasmid pEC-XK99E_AGAT_Mp

pCRII-argF was cut using Kpnl + Xbal + Asel and the DNA was purified with the "QIAquick PCR Purification Kit" (Qiagen GmbH, Hilden, Germany). The plasmid pEC-XK99E_AGAT_Mp was cut using Kpnl + Xbal, the DNA was purified with the "QIAquick PCR Purification Kit" (Qiagen GmbH, Hilden, Germany). Both eluates were mixed, the DNA fragments were ligated and the product was used to transform *E. coli.* Proper plasmid clones were identified by restriction digestion and DNA sequencing. The resulting plasmid pEC-XK99E_AGAT_Mp_argF contains the gene *argF* from *C. glutamicum* in combination with AGAT_Mp.

### Example 9: Cloning of the gene *argG* into the expression plasmid pEC-XK99E_AGAT_Mp

pCRII-argGH was cut using Xbal + Sall and a restriction fragment of 1798 bps was isolated from an agarose gel. The plasmid pEC-XK99E_AGAT_Mp was cut using Xbal + Sall, the DNA was purified with the "QIAquick PCR Purification Kit" (Qiagen GmbH, Hilden, Germany). The DNA fragments were ligated and the product was used to transform *E. coli.* Proper plasmid clones were identified by restriction digestion and DNA sequencing. The resulting plasmid pEC-XK99E_AGAT_Mp_argG contains the gene *argG* from *C. glutamicum* in combination with AGAT_Mp.

### Example 10: Chromosomal insertion of the sod promoter upstream of the *carAB* operon in ATCC13032

The enzymatic activity of the carbamoyl phosphate synthetase was increased by genomic insertion of the strong *sod*-promoter upstream of the *carAB* operon in ATCC13032. Therefore, the plasmid pK18mobsacB_Psod-carAB was constructed as follows. Plasmid pK18mobsacB was cut using EcoRI + HindIII and the linearized vector DNA (5670 bps) was cut out of an agarose gel. The DNA was extracted using the "QIAquick Gel Extraction Kit" (QIAGEN GmbH, Hilden, Germany).

For constructing the insert, three DNA fragments were created by PCR with the following pairs of primers (genomic DNA of ATCC13032 as template):

PsodcarAB-LA-F (SEQ ID NO:9) + PsodcarAB-LA-R (SEQ ID NO:10)
= left homology arm (1025 bps)
PsodcarAB-F (SEQ ID NO:11) + PsodcarAB-R (SEQ ID NO:12)
= *sod*-promoter (250 bps)
PsodcarAB-RA-F SEQ ID NO:13) + PsodcarAB-RA-R (SEQ ID NO:14)
= right homology arm (944 bps)

The product DNAs were purified using the "QIAquick PCR Purification Kit" (Qiagen GmbH, Hilden, Germany). The linearized plasmid and the PCR products were then assembled using the "NEBuilder HiFi DNA Assembly Cloning Kit" (New England BioLabs Inc., Ipswich, USA, Cat. No. E5520). Proper plasmid clones were identified by restriction digestion and DNA sequencing.

pK18mobsacB_Psod-carAB was used to integrate the strong sod-promoter upstream of the *carAB* genes into the genome of *C. glutamicum* ATCC13032. The plasmid was transformed into ATCC13032 by electroporation. Chromosomal integration (resulting from a first recombination event) was selected by plating on BHI agar supplemented with 134 g/l sorbitol, 2.5 g/l yeast extract and 25 mg/l kanamycin. The agar plates were incubated for 48 h at 33°C.

Individual colonies were transferred onto fresh agar plates (with 25 mg/l kanamycin) and incubated for 24 h at 33°C. Liquid cultures of these clones were cultivated for 24 h at 33°C in 10 ml BHI medium contained in 100 ml Erlenmeyer flasks with 3 baffles. To isolate clones that have encountered a second recombination event, an aliquot was taken from each liquid culture, suitably diluted and plated (typically 100 to 200 µl) on BHI agar supplemented with 10 % saccharose. These agar plates were incubated for 48 h at 33°C. The colonies growing on the saccharose containing agar plates were then examined for kanamycin sensitivity. To do so a toothpick was used to remove cell material from the colony and to transfer it onto BHI agar containing 25 mg/l kanamycin and onto BHI agar containing 10% saccharose. The agar plates were incubated for 60 h at 33°C. Clones that proved to be sensitive to kanamycin and resistant to saccharose were examined by PCR and DNA sequencing for the appropriate integration of the sod promoter. The resulting strain was named ATCC13032_Psod-carAB.

Table 8: List of strains

| Strain | Comment |
|---|---|
| *Escherichia coli* | |
| NEB® Stable | Commercial cloning strain (New England BioLabs Inc., Ipswich, USA) |
| MG1655 | *E. coli* reference strain (Blattner *et al.*, 1997*)) |
| *Corynebacterium glutamicum* | |
| ATCC13032 | *Corynebacterium glutamicum* wild type strain (Kinoshita *et al.*, 1957**)) |
| ATCC21831 | *C. glutamicum* variant producing L-arginine (Park *et al.*, 2014***)) |
| ATCC13032_P*sod-carAB* | Chromosomal integration of the strong sod promotor upstream of *carAB* in C. *glutamicum* ATCC13032 |
| *) Blattner et al., 1997: Blattner FR, Plunkett G 3rd, Bloch CA, Perna NT, Burland V, Riley M, Collado-Vides J, Glasner JD, Rode CK, Mayhew GF, Gregor J, Davis NW, Kirkpatrick HA, Goeden MA, Rose DJ, Mau B, Shao Y, Science. 1997 Sep 5;277(5331):1453-62. (doi: 10.1126/science.277.5331.1453) <br> **) Kinoshita S, Udaka S, Shimono M., J. Gen. Appl. Microbiol. 1957; 3(3): 193-205. <br> ***) Park et al., 2014: Park SH, Kim HU, Kim TY, Park JS, Kim SS, Lee, Nat Commun. 2014 Aug 5; 5:4618. (doi: 10.1038/ncomms5618) | |

Table 9: List of plasmids

| Plasmid | Comment |
|---|---|
| pMA-T_AGAT_Mp | Gene synthesis and cloning vector for AGAT_Mp (*Moorea producens)*; proprietary plasmid of Invitrogen/Geneart (Thermo Fisher Scientific, Waltham, USA) |
| pCR-Blunt II-TOPO | Empty plasmid for cloning PCR products (Thermo Fisher Scientific/Invitrogen, Waltham, USA) |
| pCRII-argF | pCR-Blunt II-TOPO containing the cloned gene *argF* (*C. glutamicum*) |
| pCRII-argGH | pCR-Blunt II-TOPO containing the cloned genes *argG, argH* (*C. glutamicum*) |
| pCRII-argFGH | pCR-Blunt II-TOPO containing the cloned genes *argF, argG, argH* (*C. glutamicum*) |
| pEC-XK99E | Empty *E. coli - C. glutamicum* shuttle plasmid |
| pEC-XK99E_AGAT_Mp | Expression of AGAT_Mp (*Moorea producens*) |
| pEC-XK99E_argFGH | Expression of *argF, argG, argH* (*C. glutamicum*) |
| pEC-XK99E_AGAT_Mp_argFGH | Expression of AGAT_Mp (*Moorea producens), argF, argG, argH (C. glutamicum*) |
| pEC-XK99E_AGAT_Mp_argGH | Expression of AGAT_Mp (*Moorea producens), argG, argH (C. glutamicum*) |
| pEC-XK99E_AGAT_Mp_argF | Expression of AGAT_Mp (*Moorea producens), argF (C. glutamicum*) |
| pEC-XK99E_AGAT_Mp_argG | Expression of AGAT_Mp (*Moorea producens), argG (C. glutamicum*) |
| pK18mobsacB | Empty plasmid for genome modification of *C. glutamicum* |
| pK18mobsacB_Psod-carAB | Plasmid for chromosomal integration of the strong sod promotor upstream of *carAB* in *C. glutamicum* |

**Example 11: Transformation of *C. glutamicum* strains with various expression plasmids**

The following strains of *C. glutamicum* were transformed with various plasmids:

- *C. glutamicum* ATCC13032 (Kinoshita et al., J. Gen. Appl. Microbiol. 1957; 3(3): 193-205) is a commonly used wild type strain.
- *C. glutamicum* strain ATCC21831 (Park et al., Nat Commun. 2014 Aug 5; 5:4618) synthesizes L-arginine from primary substrates like ammonia and glucose.
- ATCC13032_P*sod-carAB* is a variant of ATCC13032 with the strong sod promotor upstream of the *carAB* genes.

The strains were transformed by electroporating with various plasmids (as shown in table 10). Plasmid containing cells were selected with 25 mg/l kanamycin.

Table 10: List of plasmid-containing *C. glutamicum* strains

| Plasmid | Cloned gene | Recipient strain | Resulting strain |
|---|---|---|---|
| pEC-XK99E | none | ATCC21831 | ATCC21831/pEC-XK99E |
| pEC-XK99E_ AGAT_Mp | AGAT_Mp | ATCC21831 | ATCC21831/pEC-XK99E_AGAT_Mp |
| pEC-XK99E | none | ATCC13032 | ATCC13032/pEC-XK99E |
| pEC-XK99E_ argFGH | *argF*, *argG*, *argH* | ATCC13032 | ATCC13032/pEC-XK99E_argFGH |
| pEC-XK99E_ AGAT_Mp | AGAT_Mp | ATCC13032 | ATCC13032/pEC-XK99E_AGAT_Mp |
| pEC-XK99E_ AGAT_Mp_argG | AGAT_Mp, *argG* | ATCC13032 | ATCC13032/pEC-XK99E_AGAT_Mp_ argG |
| pEC-XK99E_ AGAT_Mp_argF | AGAT_Mp, *argF* | ATCC13032 | ATCC13032/pEC-XK99E_AGAT_Mp_argF |
| pEC-XK99E_ AGAT_Mp_argGH | AGAT_Mp, *argG*, argH | ATCC13032 | ATCC13032/pEC-XK99E_AGAT_Mp_ argGH |
| pEC-XK99E_ AGAT_Mp_ argFGH | AGAT_Mp, *argF*, *argG*, *argH* | ATCC13032 | ATCC13032/pEC-XK99E_AGAT_Mp_ argFGH |
| pEC-XK99E | none | ATCC13032_Psod-carAB | ATCC13032_PsodcarAB/pEC-XK99E |
| pEC-XK99E_ AGAT_Mp_ argFGH | AGAT_Mp, argF, argG, argH | ATCC13032_PsodcarAB | ATCC13032_PsodcarAB/pEC-XK99E_ AGAT_Mp_argFGH |

**Example 12: Impact of AGAT and substrate availability on GAA production**

Strains ATCC13032/pEC-XK99E_AGAT_Mp (carrying a gene for the AGAT enzyme from *Moorea producens*) and ATCC13032/pEC-XK99E (empty vector for control) were analyzed for their ability to produce GAA in batch cultivations using the system of Wouter Duetz (as described above). The production medium (PM) contained 40 g/l D-glucose as the main carbon source. Some batches were supplemented with L-arginine and/or glycine as indicated.

Table 11: GAA production by strains ATCC13032/pEC-XK99E and ATCC13032/pEC-XK99E_AGAT_Mp

| Strain | Supplementation | GAA |
|---|---|---|
| A TCC 13032/pEC-XK99E | none | not detectable |
| A TCC 13032/pEC-XK99E | 1.90 g/l Arg and 0.818 g/l Gly | not detectable |
| A TCC 13032/pEC-XK99E_AGAT_Mp | none | 25 mg/l |

(continued)

| Strain | Supplementation | GAA |
|---|---|---|
| ATCC13032/pEC-XK99E_AGAT_Mp | 0.818 g/l Gly | 31 mg/l |
| ATCC13032/pEC-XK99E_AGAT_Mp | 1.90 g/l Arg and 0.818 g/l Gly | 124 mg/l |

As shown in table 11, the control strain ATCC13032/pEC-XK99E could not produce GAA, even if the precursors L-arginine and glycine were provided. We conclude that it does not possess an intrinsic AGAT activity. Strain ATCC13032/pEC-XK99E_AGAT_Mp contains a polynucleotide coding for a putative AGAT from *Moorea producens*. It produced 25 mg/l GAA in unsupplemented PM. Supplementation of glycine resulted in a small increase to 31 mg/l GAA. Supplementation of glycine and L-arginine largely increased GAA production to 124 mg/l.

## Example 13: Production of GAA from primary substrates

In an industrial GAA production process, supplementation of L-arginine would be rather costly when compared to primary substrates like ammonia, urea and glucose. It would therefore be desirable to generate GAA directly from such primary substrates.

For this purpose, the L-arginine producer *C. glutamicum* ATCC21831 was transformed with pEC-XK99E_AGAT_Mp (containing the AGAT_Mp gene) and pEC-XK99E (empty vector for control). ATCC21831 was isolated as a canavanine resistant mutant and it was found to produce L-arginine. Its genome was sequenced by Park et al. (Nat Commun. 2014 Aug 5;5:4618. doi: 10.1038/ncomms5618; accession number CP007722) and the strain is publicly available from LGC Standards (LGC Standards GmbH, Wesel, Germany).

Strains ATCC21831/pEC-XK99E_AGAT_Mp and ATCC21831/pEC-XK99E were analyzed for their ability to produce GAA in batch cultivations using the system of Wouter Duetz (as described above). The production medium (PM) contained 40 g/l D-glucose as the main carbon source. Some batches were supplemented with glycine and/or L-arginine.

Table 12: GAA production by strains ATCC21831/pEC-XK99E_AGAT_Mp and ATCC21831/pEC-XK99E

| Strain | Supplementation | GAA | L-Arginine |
|---|---|---|---|
| ATCC21831/pEC-XK99E | none | not detectable | 2118 mg/l |
| ATCC21831/pEC-XK99E | 1.90 g/l Arg and 0.818 g/l Gly | not detectable | 3311 mg/l |
| ATCC21831/pEC-XK99E_AGAT_Mp | none | 166 mg/l | 2059 mg/l |
| A TCC21831/pEC-XK99E_AGAT_Mp | 0.818 g/l Gly | 476 mg/l | 1242 mg/l |

As shown in table 12, ATCC21831/pEC-XK99E did not produce GAA, even if the precursors L-arginine and glycine were present. We conclude that ATCC21831/pEC-XK99E does not have an intrinsic AGAT activity. Strain ATCC21831/pEC-XK99E_AGAT_Mp produced 166 mg/l GAA in unsupplemented PM. We conclude that the precursors L-arginine and glycine were synthesized from the primary substrates D-glucose, ammonium and urea.

When glycine was added, GAA production of ATCC21831/pEC-XK99E_AGAT_Mp raised to 476 mg/l. When compared to strain ATCC13032/pEC-XK99E_AGAT_Mp (see table 10), the L-arginine producer ATCC21831/pEC-XK99E_AGAT_Mp accumulates much more GAA when glycine is not limiting. We conclude that the capability of internally providing L-arginine improves GAA production.

## Example 14: Impact of L-arginine regeneration on GAA production

In L-arginine production strains (e.g. ATCC21831) the intermediate L-ornithine is synthesized *de novo* and further converted into L-arginine. When such a strain is provided with an AGAT, the enzyme will produce an equal molar amount of GAA and L-ornithine. The formation of L-ornithine though consumes a significant amount of the primary C- and N-sources and it thus lowers the yield of GAA.

We found that enhancement of the biosynthetic pathway leading from L-ornithine to L-arginine improves GAA production, presumably by improving the recycling of L-ornithine to L-arginine.

Various strains, derived from ATCC13032, were cultivated using the Wouter Duetz system and then analyzed for their ability to produce GAA (tables 13, 14 and 15).

Table 13: Impact of improved L-arginine regeneration on GAA production in PM comprising YE without glycine or L-arginine supplementation

| Strain | GAA |
|---|---|
| ATCC13032/pEC-XK99E | 0 mg/l |
| ATCC13032/pEC-XK99E_argFGH | 0 mg/l |
| ATCC13032_DargR/pEC-XK99E | 0 mg/l |
| ATCC13032_Psod-carAB/pEC-XK99E | 0 mg/l |
| ATCC13032/pEC-XK99E_AGAT_Mp | 25 mg/l |
| ATCC13032/pEC-XK99E_AGAT_Mp_argG | 26 mg/l |
| ATCC13032/pEC-XK99E_AGAT_Mp_argH | 24 mg/l |
| ATCC13032/pEC-XK99E_AGAT_Mp_argF | 27 mg/l |
| ATCC13032/pEC-XK99E_AGAT_Mp_argGH | 26 mg/l |
| ATCC13032/pEC-XK99E_AGAT_Mp_argFGH | 25 mg/l |
| ATCC13032_Psod-carAB/pEC-XK99E_AGAT_Mp | 43 mg/l |
| ATCC13032_Psod-carAB/pEC-XK99E_AGAT_Mp_argG | 45 mg/l |
| ATCC13032_Psod-carAB/pEC-XK99E_AGAT_Mp_argFGH | 45 mg/l |
| ATCC13032_DargR/pEC-XK99E_AGAT_Mp | 42 mg/l |
| ATCC13032_DargR/pEC-XK99E_AGAT_Mp_argG | 42 mg/l |
| ATCC13032_DargR/pEC-XK99E_AGAT_Mp_argFGH | 37 mg/l |

Table 14: Impact of improved L-arginine regeneration on GAA production in PM without YE. All cultures were supplemented with 0.82 g/l glycine

| Strain | GAA |
|---|---|
| ATCC13032/pEC-XK99E | 0 mg/l |
| ATCC13032/pEC-XK99E_argFGH | 0 mg/l |
| ATCC13032_DargR/pEC-XK99E | 0 mg/l |
| ATCC13032_Psod-carAB/pEC-XK99E | 0 mg/l |
| ATCC13032/pEC-XK99E_AGAT_Mp | 22 mg/l |
| ATCC13032/pEC-XK99E_AGAT_Mp_argG | 24 mg/l |
| ATCC13032/pEC-XK99E_AGAT_Mp_argH | 22 mg/l |
| ATCC13032/pEC-XK99E_AGAT_Mp_argF | 23 mg/l |
| ATCC13032/pEC-XK99E_AGAT_Mp_argGH | 23 mg/l |
| ATCC13032/pEC-XK99E_AGAT_Mp_argFGH | 23 mg/l |
| ATCC13032_Psod-carAB/pEC-XK99E_AGAT_Mp | 53 mg/l |
| ATCC13032_DargR/pEC-XK99E_AGAT_Mp | 40 mg/l |
| ATCC13032_DargR/pEC-XK99E_AGAT_Mp_argG | 42 mg/l |
| ATCC13032_DargR/pEC-XK99E_AGAT_Mp_argFGH | 36 mg/l |

Table 15: Impact of improved L-arginine regeneration on GAA production in PM comprising YE. All cultures were supplemented with 0.82 g/l glycine and 1.9 g/l L-arginine.

| Strain | GAA |
| --- | --- |
| A TCC 13032/pEC-XK99E | not detectable |
| A TCC13032/pEC-XK99E_argFGH | not detectable |
| ATCC13032/pEC-XK99E_AGAT_Mp | 124 mg/l |
| ATCC13032/pEC-XK99E_AGAT_Mp_argG | 136 mg/l |
| ATCC13032/pEC-XK99E_AGAT_Mp_argF | 136 mg/l |
| ATCC13032/pEC-XK99E_AGAT_Mp_argGH | 133 mg/l |
| ATCC13032/pEC-XK99E_AGAT_Mp_argFGH | 154 mg/l |
| ATCC13032_Psod-carAB/pEC-XK99E_AGAT_Mp | 156 mg/l |
| ATCC13032_Psod-carAB/pEC-XK99E_AGAT_Mp_argFGH | 171 mg/l |
| ATCC13032_DargR/pEC-XK99E_AGAT_Mp | 89 mg/l |
| ATCC13032_DargR/pEC-XK99E_AGAT_Mp_argG | 97 mg/l |
| ATCC13032_DargR/pEC-XK99E_AGAT_Mp_argFGH | 90 mg/l |

As shown in tables 13 to 15, strains lacking the AGAT gene did not produce a detectable amount of GAA.

The expression of AGAT_Mp in ATCC13032/pEC-XK99E_AGAT_Mp resulted in 124 mg/l GAA. Additional amplification of argG (strain ATCC13032/pEC-XK99E_AGAT_Mp_argG), argF (strain ATCC13032/pEC-XK99E_AGAT_Mp_argF) or argG+argH (strain ATCC13032/pEC-XK99E_AGAT_Mp_argGH) improved the production of GAA (cf. table 14).

In strain ATCC13032/pEC-XK99E_AGAT_Mp_argFGH the expression of the genes argF (coding for ornithine carbamoyltransferase), argG (coding for argininosuccinate synthetase) and argH (coding for argininosuccinate lyase) is enhanced. This further improved the production of GAA to 154 mg/l (cf. table 14).

The conversion of L-ornithine to L-citrulline, catalyzed by the ornithine carbamoyltransferase, depends on the availability of the co-substrate carbamoyl phosphate. Carbamoyl phosphate is produced by the carbamoyl phosphate synthase, which is encoded by the genes carA and carB. In strain ATCC13032_Psod-carAB/pEC-XK99E_AGAT_Mp a genomic insertion of the strong sod-promoter enhances the expression of carA and carB. When compared to ATCC13032/pEC-XK99E_AGAT_Mp, this resulted in improved GAA production (156 mg/l vs. 124 mg/l).

In strain ATCC13032_Psod-carAB/pEC-XK99E_AGAT_Mp_argFGH the improved L-ornithine conversion (overexpression of *argF*, *argG* and *argH*) was combined with the improved carbamoyl phosphate biosynthesis (overexpression of *carA* and *carB*). This combination further improved GAA production to 171 mg/l.

## Claims

1. A microorganism having an improved ability to produce L-arginine compared with the ability of the wildtype microorganism and/or having increased activities of an enzyme having the function of a carbamoylphosphate synthase compared to the respective enzymic activity in the wildtype microorganism and comprising at least one gene coding for a protein having the function of an L-arginine:glycine amidinotransferase.

2. The microorganism of claim 1, wherein the activity of an enzyme having the function of an argininosuccinate lyase is increased compared to the respective enzymic activity in the wildtype microorganism.

3. The microorganism of claim 1 or claim 2, wherein the activity of an enzyme having the function of an ornithine carbamoyltransferase is increased compared to the respective enzymic activity in the wildtype microorganism.

4. The microorganism of any of claims 1 to 3, wherein the activity of an enzyme having the function of an argininosuccinate synthetase is increased compared to the respective enzymic activity in the wildtype microorganism.

5. The microorganism of any of claims 1 to 4, wherein increased activities of the enzymes are achieved by overexpressing the genes coding for the respective enzymes.

6. The microorganism of any of claims 1 to 5, wherein the microorganism is recombinant and the gene coding for the protein having the function of an L-arginine:glycine amidinotransferase is heterologous.

7. The microorganism of any of claims 1 to 6, wherein the *argR* gene coding for the arginine responsive repressor protein ArgR is neither attenuated nor deleted.

8. The microorganism of claim 7, wherein the activity of the arginine responsive repressor protein ArgR is increased compared to the respective activity in the wildtype microorganism.

9. The microorganism of any of claims 1 to 8, wherein the arginine operon (*argCJBDFR*) is overexpressed.

10. The microorganism of any of claims 1 to 6, wherein the *argR* gene coding for the arginine responsive repressor protein ArgR is attenuated or deleted.

11. The microorganism of any of claims 1 to 6 or 10, wherein at least one or more of the genes coding for an enzyme of the biosynthetic pathway of L-arginine, comprising *gdh*, *argJ*, *argB*, *argC* and/or *argD* coding for a glutamate dehydrogenase, an ornithine acetyltransferase, an acetylglutamate kinase, an acetylglutamylphosphate reductase and for an acetylornithine aminotransferase, respectively, is overexpressed.

12. The microorganism of any of the preceding claims, wherein the gene coding for a protein having the function of an L-arginine:glycine amidinotransferase is overexpressed.

13. The microorganism of any of the preceding claims, wherein the protein having the function of an L-arginine:glycine amidinotransferase comprises an amino acid sequence which is at least 80 % homologous to the amino acid sequence according to SEQ ID NO: 4.

14. The microorganism of any of the preceding claims, wherein the microorganism belongs to the genus *Corynebacterium,* to the genus *Enterobacteriaceae* or to the genus *Pseudomonas.*

15. The microorganism of claim 14, wherein the microorganism is *Corynebacterium glutamicum.*

16. The microorganism of claim 14 wherein the microorganism is *Escherichia coli.*

17. The microorganism of claim 14 wherein the microorganism is *Pseudomonas putida.*

18. A method for the fermentative production of guanidino acetic acid (GAA), comprising the steps of a) cultivating the microorganism as defined in any of the preceding claims in a suitable medium under suitable conditions, and b) accumulating GAA in the medium to form a GAA containing fermentation broth.

19. The method of claim 18 further comprising adding glycine to the medium.

20. The method of any of claims 18 to 19 further comprising adding L-arginine to the medium.

21. The method of any of claims 18 to 20 further comprising isolating GAA from the GAA containing fermentation broth.

22. The method of any of claims 18 to 20, further comprising drying and/or granulating the GAA containing fermentation broth.

23. A microorganism as claimed in any of claims 1 to 17, further comprising a gene coding for an enzyme having the activity of a guanidinoacetate N-methyltransferase.

24. The microorganism of claim 23, wherein the gene coding for an enzyme having the activity of a guanidinoacetate N-methyltransferase is overexpressed.

25. A method for the fermentative production of creatine, comprising the steps of a) cultivating the microorganism as defined in any of claims 23 to 24 in a suitable medium under suitable conditions, and b) accumulating creatine in the medium to form a creatine containing fermentation broth.

26. The method of claim 25, further comprising isolating creatine from the creatine containing fermentation broth.

Europäisches
Patentamt

European
Patent Office

Office européen
des brevets

# EUROPEAN SEARCH REPORT

Application Number

EP 19 21 8204

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2018/079687 A1 (AJINOMOTO KK [JP]) 3 May 2018 (2018-05-03) | 1-7,9, 11,12, 14,15, 18-26 | INV. C12P7/40 C12P13/10 C12N9/10 |
| Y | * paragraphs [0090], [0091] * | 8,13,16, 17 | |
| Y | DATABASE UniProt [Online] 15 February 2017 (2017-02-15), "Glycine amidinotransferase", XP055706853, retrieved from EBI accession no. UNIPROT:A0A1D8TKD3 Database accession no. A0A1D8TKD3 * 100% identical to SEQ ID NO:4; sequence * | 13 | |
| Y | WO 2006/057450 A1 (AJINOMOTO KK [JP]; FUKUI KEITA [JP] ET AL.) 1 June 2006 (2006-06-01) * the whole document * | 8 | |
| Y | MARC F ET AL: "Characterization and kinetic mechanism of mono- and bifunctional ornithine acetyltransferases from thermophilic microorganisms", EUROPEAN JOURNAL OF BIOCHEMISTRY, WILEY-BLACKWELL PUBLISHING LTD, GB, vol. 267, no. 16, 1 August 2000 (2000-08-01), pages 5217-5226, XP002169947, ISSN: 0014-2956, DOI: 10.1046/J.1432-1327.2000.01593.X * figure 1 * | 16,17 | TECHNICAL FIELDS SEARCHED (IPC) C12P C12N |
| A | US 2006/200870 A1 (TSENG BRIAN [US]) 7 September 2006 (2006-09-07) * paragraphs [0010], [0011] * | 1-26 | |

The present search report has been drawn up for all claims

| Place of search | Date of completion of the search | Examiner |
|---|---|---|
| Berlin | 19 June 2020 | Schneider, Patrick |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                       

& : member of the same patent family, corresponding document

EPO FORM 1503 03.82 (P04C01)

**ANNEX TO THE EUROPEAN SEARCH REPORT**
**ON EUROPEAN PATENT APPLICATION NO.**

EP 19 21 8204

This annex lists the patent family members relating to the patent documents cited in the above-mentioned European search report.
The members are as contained in the European Patent Office EDP file on
The European Patent Office is in no way liable for these particulars which are merely given for the purpose of information.

19-06-2020

| Patent document cited in search report | | Publication date | Patent family member(s) | | Publication date |
|---|---|---|---|---|---|
| WO 2018079687 | A1 | 03-05-2018 | CN | 109952380 A | 28-06-2019 |
| | | | EP | 3532627 A1 | 04-09-2019 |
| | | | JP | 2019531759 A | 07-11-2019 |
| | | | US | 2019249205 A1 | 15-08-2019 |
| | | | WO | 2018079687 A1 | 03-05-2018 |
| WO 2006057450 | A1 | 01-06-2006 | AT | 526404 T | 15-10-2011 |
| | | | AU | 2005308023 A1 | 01-06-2006 |
| | | | BR | PI0518589 A2 | 25-11-2008 |
| | | | CN | 101065484 A | 31-10-2007 |
| | | | CN | 102031238 A | 27-04-2011 |
| | | | EP | 1814987 A1 | 08-08-2007 |
| | | | ES | 2375650 T3 | 05-03-2012 |
| | | | JP | 4595506 B2 | 08-12-2010 |
| | | | JP | 2006149214 A | 15-06-2006 |
| | | | KR | 20070091303 A | 10-09-2007 |
| | | | MY | 141980 A | 16-08-2010 |
| | | | PE | 20061165 A1 | 21-10-2006 |
| | | | US | 2007254345 A1 | 01-11-2007 |
| | | | WO | 2006057450 A1 | 01-06-2006 |
| US 2006200870 | A1 | 07-09-2006 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005120246 A1
- US 2011257075 A1
- US 3849250 A
- EP 1057893 A1, Kurahashi
- US 7160705 B2, Suga
- EP 3153573 A1, However, Bae
- CN 106065411 A

### Non-patent literature cited in the description

- **HUMM et al.** *Biochem. J.,* 1997, vol. 322, 771-776
- **GUTHMILLER et al.** *J Biol Chem.,* 01 July 1994, vol. 269 (26), 17556-60
- **MUENCHHOFF et al.** *FEBS Journal,* 2010, vol. 277, 3844-3860
- **SOSIO et al.** *Cell Chemical Biology,* 17 May 2018, vol. 25, 540-549
- **HUMM et al.** expressed a recombinant gene encoding human AGAT in Escherichia coli and identified cysteine-407 as an active-site residue of AGAT. *Biochem. J.,* 1997, vol. 322, 771-776
- **PARK et al.** *NATURE COMMUNICATIONS*
- **YIM et al.** *J Ind Microbiol Biotechnol,* 2011, vol. 38, 1911-1920
- **GINESY et al.** *Microbial Cell Factories,* 2015, vol. 14, 29
- **SAKANYAN et al.** *Microbiology,* 1996, vol. 142, 9-108
- **MARCHLER-BAUER A et al.** CDD/SPARCLE: functional classification of proteins via subfamily domain architectures. *Nucleic Acids Res.,* 2017, vol. 45 (D1), D200-D203
- **PISSOWOTZKI K et al.** *Mol Gen Genet,* 1991, vol. 231, 113-123
- **D'HOOGHE I et al.** *J Bacteriol,* 1997, vol. 179, 7403-7409
- **KANAOKA M et al.** *Jpn J Cancer Res,* 1987, vol. 78, 1409-1414
- **M. PATEK et al.** *Microbial Biotechnology,* 2013, vol. 6, 103-117
- **SANGER et al.** *Proceedings of the National Academy of Sciences USA,* 1977, vol. 74, 5463-5467
- **LEAO T ; CASTELÃO G ; KOROBEYNIKOV A ; MONROE EA ; PODELL S ; GLUKHOV E ; ALLEN EE ; GERWICK WH ; GERWICK L.** *Proc Natl Acad Sci U S A.,* 21 March 2017, vol. 114 (12), 3198-3203
- **BLATTNER FR ; PLUNKETT G 3RD ; BLOCH CA ; PERNA NT ; BURLAND V ; RILEY M ; COLLADO-VIDES J ; GLASNER JD ; RODE CK ; MAYHEW GF.** *Science,* 05 September 1997, vol. 277 (5331), 1453-62
- **KINOSHITA S ; UDAKA S ; SHIMONO M.** *J. Gen. Appl. Microbiol.,* 1957, vol. 3 (3), 193-205
- **PARK SH ; KIM HU ; KIM TY ; PARK JS ; KIM SS, LEE.** *Nat Commun.,* 05 August 2014, vol. 5, 4618
- **KINOSHITA et al.** *J. Gen. Appl. Microbiol.,* 1957, vol. 3 (3), 193-205
- **PARK et al.** *Nat Commun.,* 05 August 2014, vol. 5, 4618

SEQUENCE LISTING

<110> Evonik Operations GmbH

<120> Method for producing guanidinium acetate

<130> 000

<160> 14

<170> PatentIn version 3.5

<210> 1
<211> 1146
<212> DNA
<213> Moorea producens strain PAL-8-15-08-1


<220>
<221> CDS
<222> (1)..(1143)
<223> Genbank accession Number WP_070390602


<400> 1
```
atg tcg gaa aaa att gtt aat tcc tgg aat gaa tgg gat gaa ttg gaa      48
Met Ser Glu Lys Ile Val Asn Ser Trp Asn Glu Trp Asp Glu Leu Glu
1               5                   10                  15

gaa atg gtg gta gga att gca gac tat gct agc ttt gaa cca aaa gaa      96
Glu Met Val Val Gly Ile Ala Asp Tyr Ala Ser Phe Glu Pro Lys Glu
                20                  25                  30

cca ggg aat cat ccg aaa tta aga aat caa aat tta gcg gaa atc att     144
Pro Gly Asn His Pro Lys Leu Arg Asn Gln Asn Leu Ala Glu Ile Ile
            35                  40                  45

cct ttc ccc agt gga cct aaa gac cct aaa gtc ctt gaa aaa gct aat     192
Pro Phe Pro Ser Gly Pro Lys Asp Pro Lys Val Leu Glu Lys Ala Asn
        50                  55                  60

gaa gaa tta aat gga ctg gct tat tta tta aaa gac cac gat gtg ata     240
Glu Glu Leu Asn Gly Leu Ala Tyr Leu Leu Lys Asp His Asp Val Ile
65                  70                  75                  80

gta aga aga ccc gaa aaa att gat ttt act aaa tct cta aaa aca cct     288
Val Arg Arg Pro Glu Lys Ile Asp Phe Thr Lys Ser Leu Lys Thr Pro
                85                  90                  95

tac ttt gaa gtt gca aat caa tac tgt gga gtc tgt cct cgg gat gtc     336
Tyr Phe Glu Val Ala Asn Gln Tyr Cys Gly Val Cys Pro Arg Asp Val
                100                 105                 110

atg att acc ttt ggg aat gaa atc atg gaa gcg act atg tcg aag aga     384
Met Ile Thr Phe Gly Asn Glu Ile Met Glu Ala Thr Met Ser Lys Arg
                115                 120                 125

gct aga ttt ttt gaa tac tta cct tac cgg aaa ttg gtc tat gaa tat     432
Ala Arg Phe Phe Glu Tyr Leu Pro Tyr Arg Lys Leu Val Tyr Glu Tyr
        130                 135                 140

tgg aat aaa gac gag cat atg att tgg aat gct gcg cct aaa ccg act     480
Trp Asn Lys Asp Glu His Met Ile Trp Asn Ala Ala Pro Lys Pro Thr
145                 150                 155                 160
```

```
atg cag gat agt atg tat cta gag aat ttc tgg gag ctg tct tta gaa          528
Met Gln Asp Ser Met Tyr Leu Glu Asn Phe Trp Glu Leu Ser Leu Glu
            165             170             175

gaa cga ttt aag cgt atg cat gat ttt gaa ttt tgt att aca caa gat          576
Glu Arg Phe Lys Arg Met His Asp Phe Glu Phe Cys Ile Thr Gln Asp
            180             185             190

gaa gta att ttt gat gcg gct gac tgt agc aga tta gga aag gat ata          624
Glu Val Ile Phe Asp Ala Ala Asp Cys Ser Arg Leu Gly Lys Asp Ile
            195             200             205

tta gtt cag gaa tcg atg aca aca aat aga aca gga att cgg tgg tta          672
Leu Val Gln Glu Ser Met Thr Thr Asn Arg Thr Gly Ile Arg Trp Leu
210             215             220

aaa aag cac cta gaa cca aga gga ttt cgg gtt cac cct gtt cat ttt          720
Lys Lys His Leu Glu Pro Arg Gly Phe Arg Val His Pro Val His Phe
225             230             235             240

ccc ctt gat ttt ttc ccc tca cac att gac tgt acg ttt gtt cct ttg          768
Pro Leu Asp Phe Phe Pro Ser His Ile Asp Cys Thr Phe Val Pro Leu
                245             250             255

cga cca ggt ctt att ttg aca aac cct gaa aga cct ata cgg gaa gag          816
Arg Pro Gly Leu Ile Leu Thr Asn Pro Glu Arg Pro Ile Arg Glu Glu
            260             265             270

gag gag aag att ttt aaa gag aat ggc tgg gag ttg atc aca gtt cct          864
Glu Glu Lys Ile Phe Lys Glu Asn Gly Trp Glu Leu Ile Thr Val Pro
            275             280             285

caa ccg act tgc tcg aat gat gaa atg cca atg ttt tgc cag tcc agt          912
Gln Pro Thr Cys Ser Asn Asp Glu Met Pro Met Phe Cys Gln Ser Ser
290             295             300

aag tgg ttg tca atg aat gtt ctg agt ata tca ccg aca aag gtt atc          960
Lys Trp Leu Ser Met Asn Val Leu Ser Ile Ser Pro Thr Lys Val Ile
305             310             315             320

tgt gag gaa aga gaa aaa cct ctc caa gaa ttg ttg gat aag cat gga         1008
Cys Glu Glu Arg Glu Lys Pro Leu Gln Glu Leu Leu Asp Lys His Gly
            325             330             335

ttt gag gtt ttt cct tta ccc ttt aga cat gtc ttt gaa ttt ggg ggg         1056
Phe Glu Val Phe Pro Leu Pro Phe Arg His Val Phe Glu Phe Gly Gly
            340             345             350

tct ttt cat tgt gca act tgg gat att cgc cga aaa ggt gag tgt gaa         1104
Ser Phe His Cys Ala Thr Trp Asp Ile Arg Arg Lys Gly Glu Cys Glu
            355             360             365

gat tat tta cca aat tta aac tat caa ccg att tgt ggt taa             1146
Asp Tyr Leu Pro Asn Leu Asn Tyr Gln Pro Ile Cys Gly
            370             375             380


<210>    2
<211>    381
<212>    PRT
<213>    Moorea producens strain PAL-8-15-08-1
```

20

<400> 2

Met Ser Glu Lys Ile Val Asn Ser Trp Asn Glu Trp Asp Glu Leu Glu
1               5                   10                  15

Glu Met Val Val Gly Ile Ala Asp Tyr Ala Ser Phe Glu Pro Lys Glu
            20                  25                  30

Pro Gly Asn His Pro Lys Leu Arg Asn Gln Asn Leu Ala Glu Ile Ile
            35                  40                  45

Pro Phe Pro Ser Gly Pro Lys Asp Pro Lys Val Leu Glu Lys Ala Asn
        50                  55                  60

Glu Glu Leu Asn Gly Leu Ala Tyr Leu Leu Lys Asp His Asp Val Ile
65                  70                  75                  80

Val Arg Arg Pro Glu Lys Ile Asp Phe Thr Lys Ser Leu Lys Thr Pro
            85                  90                  95

Tyr Phe Glu Val Ala Asn Gln Tyr Cys Gly Val Cys Pro Arg Asp Val
            100                 105                 110

Met Ile Thr Phe Gly Asn Glu Ile Met Glu Ala Thr Met Ser Lys Arg
            115                 120                 125

Ala Arg Phe Phe Glu Tyr Leu Pro Tyr Arg Lys Leu Val Tyr Glu Tyr
            130                 135                 140

Trp Asn Lys Asp Glu His Met Ile Trp Asn Ala Ala Pro Lys Pro Thr
145                 150                 155                 160

Met Gln Asp Ser Met Tyr Leu Glu Asn Phe Trp Glu Leu Ser Leu Glu
            165                 170                 175

Glu Arg Phe Lys Arg Met His Asp Phe Glu Phe Cys Ile Thr Gln Asp
            180                 185                 190

Glu Val Ile Phe Asp Ala Ala Asp Cys Ser Arg Leu Gly Lys Asp Ile
            195                 200                 205

Leu Val Gln Glu Ser Met Thr Thr Asn Arg Thr Gly Ile Arg Trp Leu
            210                 215                 220

Lys Lys His Leu Glu Pro Arg Gly Phe Arg Val His Pro Val His Phe
225                 230                 235                 240

Pro Leu Asp Phe Phe Pro Ser His Ile Asp Cys Thr Phe Val Pro Leu

                        245                    250                        255

        Arg Pro Gly Leu Ile Leu Thr Asn Pro Glu Arg Pro Ile Arg Glu Glu
                    260                    265                    270

        Glu Glu Lys Ile Phe Lys Glu Asn Gly Trp Glu Leu Ile Thr Val Pro
                    275                    280                    285

        Gln Pro Thr Cys Ser Asn Asp Glu Met Pro Met Phe Cys Gln Ser Ser
                290                    295                    300

        Lys Trp Leu Ser Met Asn Val Leu Ser Ile Ser Pro Thr Lys Val Ile
        305                    310                    315                    320

        Cys Glu Glu Arg Glu Lys Pro Leu Gln Glu Leu Leu Asp Lys His Gly
                    325                    330                    335

        Phe Glu Val Phe Pro Leu Pro Phe Arg His Val Phe Glu Phe Gly Gly
                    340                    345                    350

        Ser Phe His Cys Ala Thr Trp Asp Ile Arg Arg Lys Gly Glu Cys Glu
                    355                    360                    365

        Asp Tyr Leu Pro Asn Leu Asn Tyr Gln Pro Ile Cys Gly
                370                    375                    380


        <210>  3
        <211>  1248
        <212>  DNA
        <213>  synthetic DNA


        <220>
        <221>  RBS
        <222>  (49)..(52)
        <223>  Shine-Dalgarno-Sequenz AGGA

        <220>
        <221>  CDS
        <222>  (58)..(1200)
        <223>  open reading frame of optimized  AGAT_Mp; protein is identical to
               genbank accession number WP_070390602

        <400>  3
        cgtctctgtg gataactgag cggataagtt cctagtacgc gtgcgagcag gaagaac          57

        atg agc gag aaa att gtg aac agc tgg aat gaa tgg gat gaa ctg gaa          105
        Met Ser Glu Lys Ile Val Asn Ser Trp Asn Glu Trp Asp Glu Leu Glu
        1               5                   10                  15

        gaa atg gtt gtt ggt att gca gat tat gca agc ttt gaa ccg aaa gaa          153
        Glu Met Val Val Gly Ile Ala Asp Tyr Ala Ser Phe Glu Pro Lys Glu
                    20                  25                  30

```
ccg ggt aat cat ccg aaa ctg cgt aat cag aat ctg gca gaa att att        201
Pro Gly Asn His Pro Lys Leu Arg Asn Gln Asn Leu Ala Glu Ile Ile
        35              40              45

ccg ttt ccg agc ggt ccg aaa gat ccg aaa gtt ctg gaa aaa gca aat        249
Pro Phe Pro Ser Gly Pro Lys Asp Pro Lys Val Leu Glu Lys Ala Asn
    50              55              60

gaa gaa ctg aat ggt ctg gcc tat ctg ctg aaa gat cat gat gtt att        297
Glu Glu Leu Asn Gly Leu Ala Tyr Leu Leu Lys Asp His Asp Val Ile
65              70              75              80

gtt cgc cgt ccg gaa aaa atc gac ttt acc aaa agc ctg aaa acc ccg        345
Val Arg Arg Pro Glu Lys Ile Asp Phe Thr Lys Ser Leu Lys Thr Pro
                85              90              95

tat ttc gaa gtt gcc aat cag tat tgt ggt gtt tgt ccg cgt gat gtt        393
Tyr Phe Glu Val Ala Asn Gln Tyr Cys Gly Val Cys Pro Arg Asp Val
            100             105             110

atg att acc ttt ggc aac gaa att atg gaa gcc acc atg agc aaa cgt        441
Met Ile Thr Phe Gly Asn Glu Ile Met Glu Ala Thr Met Ser Lys Arg
        115             120             125

gcc cgt ttt ttt gaa tat ctg ccg tat cgt aaa ctg gtg tat gag tat        489
Ala Arg Phe Phe Glu Tyr Leu Pro Tyr Arg Lys Leu Val Tyr Glu Tyr
        130             135             140

tgg aac aaa gat gag cat atg atc tgg aat gca gca ccg aaa ccg acc        537
Trp Asn Lys Asp Glu His Met Ile Trp Asn Ala Ala Pro Lys Pro Thr
145             150             155             160

atg cag gat agc atg tat ctg gaa aac ttt tgg gaa ctg agc ctg gaa        585
Met Gln Asp Ser Met Tyr Leu Glu Asn Phe Trp Glu Leu Ser Leu Glu
                165             170             175

gaa cgt ttt aaa cgt atg cac gat ttt gag ttt tgc atc acc cag gat        633
Glu Arg Phe Lys Arg Met His Asp Phe Glu Phe Cys Ile Thr Gln Asp
            180             185             190

gaa gtg att ttt gat gca gca gat tgt agc cgt ctg ggt aaa gat att        681
Glu Val Ile Phe Asp Ala Ala Asp Cys Ser Arg Leu Gly Lys Asp Ile
        195             200             205

ctg gtt caa gaa agc atg acc acc aat cgt acc ggt att cgt tgg ctg        729
Leu Val Gln Glu Ser Met Thr Thr Asn Arg Thr Gly Ile Arg Trp Leu
        210             215             220

aaa aaa cat ctg gaa ccg cgt ggt ttt cgt gtt cat ccg gtt cat ttt        777
Lys Lys His Leu Glu Pro Arg Gly Phe Arg Val His Pro Val His Phe
225             230             235             240

ccg ctg gat ttt ttt ccg agc cat att gat tgt acc ttt gtt ccg ctg        825
Pro Leu Asp Phe Phe Pro Ser His Ile Asp Cys Thr Phe Val Pro Leu
            245             250             255

cgt ccg ggt ctg att ctg acc aat ccg gaa cgt ccg att cgt gaa gaa        873
Arg Pro Gly Leu Ile Leu Thr Asn Pro Glu Arg Pro Ile Arg Glu Glu
            260             265             270

gaa gag aaa atc ttc aaa gag aat ggc tgg gag ctg att acc gtt ccg        921
Glu Glu Lys Ile Phe Lys Glu Asn Gly Trp Glu Leu Ile Thr Val Pro
        275             280             285
```

23

```
cag ccg acc tgt agc aat gat gaa atg ccg atg ttt tgt cag agc agc        969
Gln Pro Thr Cys Ser Asn Asp Glu Met Pro Met Phe Cys Gln Ser Ser
    290             295             300

aaa tgg ctg agc atg aat gtt ctg agc att agc ccg acc aaa gtt att       1017
Lys Trp Leu Ser Met Asn Val Leu Ser Ile Ser Pro Thr Lys Val Ile
305             310             315             320

tgt gaa gaa cgt gaa aaa ccg ctg caa gaa ctg ctg gat aaa cat ggt       1065
Cys Glu Glu Arg Glu Lys Pro Leu Gln Glu Leu Leu Asp Lys His Gly
            325             330             335

ttt gaa gtg ttt ccg ctg ccg ttt cgt cat gtt ttt gaa ttt ggt ggt      1113
Phe Glu Val Phe Pro Leu Pro Phe Arg His Val Phe Glu Phe Gly Gly
            340             345             350

agc ttt cat tgt gcc acc tgg gat att cgt cgt aaa ggt gaa tgt gaa      1161
Ser Phe His Cys Ala Thr Trp Asp Ile Arg Arg Lys Gly Glu Cys Glu
            355             360             365

gat tat ctg ccg aat ctg aat tat cag ccg att tgt ggt taataagacg       1210
Asp Tyr Leu Pro Asn Leu Asn Tyr Gln Pro Ile Cys Gly
    370             375             380

tccgcgaggg ccgtgttgcc ggtttcttca gagagacg                            1248
```

```
<210>  4
<211>  381
<212>  PRT
<213>  synthetic DNA

<400>  4
```

```
Met Ser Glu Lys Ile Val Asn Ser Trp Asn Glu Trp Asp Glu Leu Glu
1               5               10              15

Glu Met Val Val Gly Ile Ala Asp Tyr Ala Ser Phe Glu Pro Lys Glu
            20              25              30

Pro Gly Asn His Pro Lys Leu Arg Asn Gln Asn Leu Ala Glu Ile Ile
            35              40              45

Pro Phe Pro Ser Gly Pro Lys Asp Pro Lys Val Leu Glu Lys Ala Asn
    50              55              60

Glu Glu Leu Asn Gly Leu Ala Tyr Leu Leu Lys Asp His Asp Val Ile
65              70              75              80

Val Arg Arg Pro Glu Lys Ile Asp Phe Thr Lys Ser Leu Lys Thr Pro
            85              90              95

Tyr Phe Glu Val Ala Asn Gln Tyr Cys Gly Val Cys Pro Arg Asp Val
            100             105             110
```

```
Met Ile Thr Phe Gly Asn Glu Ile Met Glu Ala Thr Met Ser Lys Arg
        115                 120             125

Ala Arg Phe Phe Glu Tyr Leu Pro Tyr Arg Lys Leu Val Tyr Glu Tyr
        130                 135             140

Trp Asn Lys Asp Glu His Met Ile Trp Asn Ala Ala Pro Lys Pro Thr
145                 150                 155                 160

Met Gln Asp Ser Met Tyr Leu Glu Asn Phe Trp Glu Leu Ser Leu Glu
                165                 170                 175

Glu Arg Phe Lys Arg Met His Asp Phe Glu Phe Cys Ile Thr Gln Asp
            180                 185                 190

Glu Val Ile Phe Asp Ala Ala Asp Cys Ser Arg Leu Gly Lys Asp Ile
            195                 200                 205

Leu Val Gln Glu Ser Met Thr Thr Asn Arg Thr Gly Ile Arg Trp Leu
        210                 215                 220

Lys Lys His Leu Glu Pro Arg Gly Phe Arg Val His Pro Val His Phe
225                 230                 235                 240

Pro Leu Asp Phe Phe Pro Ser His Ile Asp Cys Thr Phe Val Pro Leu
                245                 250                 255

Arg Pro Gly Leu Ile Leu Thr Asn Pro Glu Arg Pro Ile Arg Glu Glu
            260                 265                 270

Glu Glu Lys Ile Phe Lys Glu Asn Gly Trp Glu Leu Ile Thr Val Pro
        275                 280                 285

Gln Pro Thr Cys Ser Asn Asp Glu Met Pro Met Phe Cys Gln Ser Ser
        290                 295                 300

Lys Trp Leu Ser Met Asn Val Leu Ser Ile Ser Pro Thr Lys Val Ile
305                 310                 315                 320

Cys Glu Glu Arg Glu Lys Pro Leu Gln Glu Leu Leu Asp Lys His Gly
            325                 330                 335

Phe Glu Val Phe Pro Leu Pro Phe Arg His Val Phe Glu Phe Gly Gly
            340                 345                 350

Ser Phe His Cys Ala Thr Trp Asp Ile Arg Arg Lys Gly Glu Cys Glu
        355                 360                 365
```

```
Asp Tyr Leu Pro Asn Leu Asn Tyr Gln Pro Ile Cys Gly
    370             375             380
```

```
<210>   5
<211>   30
<212>   DNA
<213>   synthetic oligonucleotide

<400>   5
ccgttaactg ccgagacaat cgcataaagg                                    30


<210>   6
<211>   34
<212>   DNA
<213>   synthetic oligonucleotide

<400>   6
ctcctaggct aatattctta cctcggctgg ttgg                               34


<210>   7
<211>   27
<212>   DNA
<213>   synthetic oligonucleotide

<400>   7
ccgttaacac ggctggcaag gaactta                                       27


<210>   8
<211>   41
<212>   DNA
<213>   synthetic oligonucleotide

<400>   8
gccctaggtc aatattacag gccataaact aatgcttatc g                       41


<210>   9
<211>   46
<212>   DNA
<213>   synthetic oligonucleotide

<400>   9
ggaaacagct atgacatgat tacgcggtta tcgcggaatc cgtatg                  46


<210>   10
<211>   25
<212>   DNA
<213>   PsodcarAB-LA-R

<400>   10
ttaagcgttt tgtgcaactc cgtct                                         25


<210>   11
<211>   50
<212>   DNA
<213>   synthetic oligonucleotide
```

26

```
<400>  11
agacggagtt gcacaaaacg cttaaaccct acttagctgc caattattcc          50


<210>  12
<211>  50
<212>  DNA
<213>  synthetic oligonucleotide

<400>  12
ggtaggtggt ggtgtcttta ctcatgggta aaaaatcctt tcgtaggttt          50


<210>  13
<211>  25
<212>  DNA
<213>  synthetic oligonucleotide

<400>  13
atgagtaaag acaccaccac ctacc                                     25


<210>  14
<211>  46
<212>  DNA
<213>  synthetic oligonucleotide

<400>  14
gttgtaaaac gacggccagt gccaccggtg atgtggttct tcactg              46
```